# EUROPEAN PATENT APPLICATION

(11) **EP 0 896 816 A1**
(43) Date of publication of application: **17.02.1999**
(21) Application number: 97905406.1
(22) Date of filing: 26.02.1997
(51) Int. Cl.: A61K 9/127, A61K 47/24, A61K 31/557

(54) **LIPOSOME AND LIPOSOME DISPERSION**

(30) Priority: 26.02.1996 JP 37688/96
(71) Applicant: DAIICHI PHARMACEUTICAL CO., LTD., Chuo-ku, Tokyo 103 (JP)
(72) Inventor: YAMAUCHI, Hitoshi, Daiichi Pharmaceutical Co., Ltd, Tokyo 134 (JP); MORITA, Hiromi, Daiichi Pharmaceutical Co., Ltd., Tokyo 134 (JP); KIKUCHI, Hiroshi, Daiichi Pharmaceutical Co., Ltd., Tokyo 134 (JP)
(74) Representative: Kinzebach, Werner, Dr.
(86) International application number: JP9700561
(87) International publication number: WO9730696

(57) **Abstract**

This invention aims at improving stability of drugs which have poor stability in aqueous solution, and the stability of such drugs are improved by incorporating the drugs in liposomes which have a sphingolipid as the main component of the liposomal membrane-constituting lipids.

## Description

### TECHNICAL FIELD

This invention relates to liposomes and a dispersion of the liposomes. The object of this invention is to improve stability of drugs which have poor stability in their aqueous solution, by incorporating them in liposomes containing a sphingolipid as the main component of the liposomal membrane-constituting lipids.

### BACKGROUND ART

In order to exert therapeutic effect of a drug by supplying it to the clinical field stably, it is necessary that pharmaceutical preparations containing the drug are stable over a prolonged period of time. Particularly, in the case of prostanoid drugs, their stability in aqueous solution is extremely low excluding some compounds thereof, so that they are made into freeze-dried preparations which are dissolved prior to use as injections (*Pharmacia*, vol. 23, no. 12, p. 1237 (1987)).

On the other hand, with the progress in the techniques on drug delivery system in recent years, a pharmaceutical preparation in the form of suspension in which prostaglandin E₁ is dissolved in lipid microspheres, which is an oil-in-water type emulsion preparation prepared by emulsifying soybean oil with a phospholipid, has been developed, but its stability is not sufficient (*Pharmaceutical Research*, vol. 5, no. 8, p. 482 (1988)).

As an example of efforts to stabilize prostaglandin E₁ making use of liposomes composed of lipid bilayer, there is a freeze-dried liposome preparation which has dimyristoyl phosphatidylcholine as the main component and contains arachidonic acid metabolites and the like as the active ingredient (JP-A-61-100518, the term "JP-A" as used herein means an "unexamined published Japanese patent application"), but, though this is a freeze drying type preparation, it cannot be said that sufficient stability was obtained.

Though there are methods developed with the aim of improving uptake ratio of drugs and improving stability of pharmaceutical preparations by providing a pH gradient inside the lipid bilayer (JP-A-4-297413 and JP-A-4-338322), they have a problem in terms of practical use because of complex handling.

Freeze-dried liposomes containing prostaglandin have been reported (JP-W-6-507417, the term "JP-W" as used herein means an "unexamined published International Japanese patent application"), but it merely uses egg yolk lecithin in Examples as a liposome-constituting lipid. Also, with regard to the pH of the liposome preparation, it describes that the pH values inside and outside of the membrane are the same but preferably about less than 5.0. However, in spite of the freeze-dried state, the stability of prostaglandin is only one year even under a low temperature (6°C) storage in a refrigerator, so that it cannot be said that the stability is sufficient.

The object of the present invention is to provide liposomes and a liposomal dispersion in which stability of drugs which have poor stability in their aqueous solution is improved.

### DISCLOSURE OF THE INVENTION

As a result of extensive investigation to resolve the aforementioned problems, the present inventors have found that the stability of drugs which have poor stability in their aqueous solution becomes markedly excellent when they are incorporated in liposomes prepared using a specified lipid.

That is, it was found that the stability of drugs which have poor stability in their aqueous solution becomes markedly excellent when they are incorporated in liposomes prepared using a sphingolipid as the specified lipid.

Accordingly, the present invention relates to the following items (A) to (Q).
(A) A liposomal preparation in which a drug is incorporated in liposomes comprised of a sphingolipid as the main component of the membrane-constituting lipids.
(B) The liposomal preparation according to the above item (A) wherein the sphingolipid is a sphingophospholipid.
(C) The liposomal preparation according to the above item (A) wherein the sphingophospholipid is sphingomyelin.
(D) A liposomal dispersion in which the liposomes of any one of the aforementioned items (A) to (C) are dispersed.
(E) The liposomal dispersion according to the above item (D) wherein dispersion medium of the dispersion is an aqueous solvent.
(F) The liposomal dispersion according to the above item (E) wherein the aqueous solvent is a buffer solution.
(G) The liposomal dispersion according to the above item (F) wherein the buffer solution is a citrate buffer.
(H) The liposomal dispersion according to the above item (G) wherein concentration of the citrate buffer is from 0.05 to 0.2% by weight.
(I) The liposomal dispersion according to any one of the aforementioned items (D) to (H) wherein concentration of the sphingolipid is 10 mM or more.
(J) The liposomal dispersion according to the above item (I) wherein concentration of the sphingolipid is from 10 to 40 mM.
(K) The liposomal dispersion according to any one of the aforementioned items (D) to (J) wherein pH of the dispersion is from 5 to 7.
(L) The liposomal dispersion according to any one of the aforementioned items (D) to (K) wherein it can be preserved under dispersed state, dried state or frozen state.
(M) The liposomal preparation or liposomal dispersion according to any one of the aforementioned items (A) to (L) wherein the drug is a drug which has poor stability in its aqueous solution.
(N) The liposomal preparation or liposomal dispersion according to any one of the aforementioned items (A) to (L) wherein the drug is an arachidonic acid metabolite or a derivative thereof.
(O) The liposomal preparation or liposomal dispersion according to any one of the aforementioned items (A) to (L) wherein the drug is a prostaglandin.
(P) The liposomal preparation or liposomal dispersion according to the above item (O) wherein the prostaglandin is prostaglandin E₁.
(Q) A liposomal dispersion prepared by dispersing liposomes which are comprised of sphingomyelin as the main component of the membrane-constituting lipids and incorporate prostaglandin E₁ therein, wherein concentration of sphingomyelin is from 10 to 40 mM, the dispersion medium is a citrate buffer of from 0.05 to 0.2% by weight, the pH is from 5 to 7 and mean particle size of the liposomes is from 100 to 250 nm.

The present invention is described in the following.

According to the present invention, the term "liposomes in which a drug is incorporated" means liposomes in which a drug is incorporated in the inside and/or on the surface of the lipid bilayer of the liposomes.

The sphingolipid as the main component of the liposomal membrane-constituting lipids according to the present invention is a lipid which has sphingosine as the nucleus of its structure and can be divided roughly into sphingophospholipids and sphingoglycolipids. According to the present invention, its kinds and origins are not particularly limited with the proviso that it can form liposomes.

The liposomes of the present invention may be produced using a sphingophospholipid alone, a sphingoglycolipid alone or a mixture of the sphingophospholipid and sphingoglycolipid, as the main component of the constituting lipids of the membrane. In producing the liposomes, cholesterol, cholestanol and the like sterols may be added as liposomal membrane stabilizing agent, and α-tocopherol or the like as an antioxidant.

According to the present invention, a sphingophospholipid is desirable among sphingolipid as the liposomal membrane-constituting lipid. Examples of the sphingophospholipid include sphingomyelin, ceramide phosphorylethanolamine, ceramide phosphorylglycerol, ceramide phosphorylglycerol phosphate, 1,2-dimyristoylamido-1,2-deoxyphoaphatidylcholine and the like, of which sphingomyelin is preferred. The origin of sphingomyelin, though not particularly limited, include bovine brain, egg yolk, microorganisms and the like.

The dispersion medium of the liposomal dispersion of the present invention should not particularly be limited, with the proviso that it is an aqueous solvent, and its examples include water, various buffer solutions, various sugar solutions, water-soluble organic solvents and the like. These aqueous solvents may be used as a mixture thereof. Examples of the buffer solution include citrate buffer, phosphate buffer, acetate buffer, lactate buffer and the like, of which citrate buffer is preferred.

The buffer solution can be prepared by adding a buffering agent to water. The buffering agent should not particularly be limited, with the proviso that it can adjust pH, and its examples include citric acid, phosphoric acid, acetic acid, lactic acid and the like, of which citric acid is preferred. Also, though it should not be limited particularly, concentration of the buffering agent may be preferably 0.05% by weight or more, more preferably from 0.05 to 0.2% by weight.

As the sugar solution, solutions of maltose, lactose, galactose, sucrose and the like saccharides can be exemplified.

As the water-soluble organic solvent, glycerol, propylene glycol, polyethylene glycol and the like polyhydric alcohols can be exemplified.

The dispersion of liposomes of the present invention has high drug retaining ratio and shows markedly excellent storage stability of the drugs incorporated in the liposomes. The term concentration of sphingolipid in the liposomal dispersion of the present invention means a concentration as the membrane-constituting component of the liposomes, and such a concentration should not particularly be limited but is preferably 10 mM or more, more preferably within the range of from 10 to 40 mM, when the dispersion is preserved as such.

The liposomal dispersion of the present invention can be stored by various methods. That is, the dispersion can be preserved as such or by freezing it. Alternatively, the dispersion may be preserved by further freeze-drying or spray-drying it. When the liposomal dispersion is stored in its freeze-dried or spray-dried form, the concentration of sphingomyelin may be adjusted to 10 mM or less.

With regard to the pH of the liposomal dispersion of the present invention, it is desirable that the pH value is within the range of from 5 to 7.

When the dispersion is preserved as it is, a liposomal dispersion adjusted to pH 5 to 6 may be prepared or, when the liposomal dispersion is preserved by freezing it, a liposomal dispersion adjusted to pH 5 to 6 may be prepared and then frozen. Also, when the liposomal dispersion is preserved by freeze-drying or spray-drying it, a liposomal dispersion adjusted to pH 6 to 7 may be prepared and then freeze-dried or spray-dried.

The liposomes and liposomal dispersion of the present invention can be prepared using a sphingolipid in accordance with the known method (*Annual Review of Biophysics and Bioengineering*, vol. 9, p. 467, 1980).

In producing the liposomes, cholesterol, cholestanol and the like sterols may be added as membrane stabilizing agent, and α-tocopherol and the like as antioxidants.

The liposomes which have a sphingolipid as the main component of the liposomal membrane-constituting lipids and contain a drug of interest, and a dispersion of the liposomes, may be produced by dissolving the drug in an organic solvent together with the lipid component, making the mixture dried, and then mixing it with the aqueous solvent to make a dispersion of liposomes, or by adding an aqueous solution of the drug to a dried lipid film of the lipid component prepared in advance, allowing the mixture to stand for a predetermined period of time, preferably by heating it above the phase transition temperature (Tc) of the membrane, and then cooling it spontaneously.

Mean particle size of the liposomes of the present invention should not particularly be limited but is preferably from 20 to 500 nm, more preferably from 100 to 250 nm. Liposomes having such a particle size hardly receive inactivation in the lungs.

The liposomes or liposomal dispersion of the present invention can be made into optional pharmaceutical preparations which may be either an oral administration preparation or a parenteral administration preparation. Examples of the oral administration preparation include capsules, granules, tablets, syrups, troches, lemonades, dry syrups and the like, and examples of the parenteral administration preparation include injections, external preparations (for example, suppositories, ointments, eye ointments, plasters, poultices, eye drops, liniments, lotions and the like), inhalations (aerosols) and the like.

Formulation of these preparations can be made from a liposomal dispersion or any of dispersed, dried and frozen conditions of liposomes, and the condition may be selected in response to the type of each preparation. Also, the formulation can generally be carried out by adding appropriate additives.

Preferred examples of the preparations of the liposomes or liposomal dispersion of the present invention are parenteral administration preparations of which injections are particularly preferred.

In producing injections, a stabilizing agent, a tonicity agent, a pH adjusting agent and the like additives may be added to the liposomal dispersion of the present invention. Though it should not particularly be limited, examples of the stabilizing agent include ethylenediaminetetraacetic acid, tocopherol, ascorbic acid and the like.

The tonicity agent is used for effecting isotonization with blood or body fluid, and its examples include saccharides, polyhydric alcohols, sodium chloride and the like. Also, examples of the pH adjusting agent include hydrochloric acid, sodium hydroxide and the like, though should not particularly be limited thereto, provided that they are acids or alkalis which are harmless to the human body.

In addition, according to the present invention, liposomes under a dried condition can be used by dissolving (dispersing) them at the time of their use as a matter of course when used, for example, as injections, and they may be in the form of a kit.

The drug to be incorporated in the liposomes and liposomal dispersion of the present invention should not particularly be limited, but it is suitable for incorporating a drug which has poor stability in its aqueous solution. Examples of the drug having poor stability in aqueous solution include prostaglandin (PG), thromboxane, leukotriene, 6-ketoPGE₁ derivatives and the like arachidonic acids and derivatives thereof, interferon, interleukin, tumor necrosis factor (TNF), epidermal growth factor (EGF), nerve growth factor (NGF), hepatocyte growth factor (HGF), atrial natiuretic peptide (ANP), erythropoietin and the like physiologically active peptides, steroids and the like hormones, cytosine arabinoside, daunorubicin, doxoribicin, aclarubicin, 4-O-tetrahydropyranyl-adriamycin, 4-epiadriamycin, 4-demethoxydaunomycin, mitomycin C, bleomycin, methotrexate, camptothecin and derivatives thereof and the like carcinostatic agents, ampicillin, cefalexin, gentamicin, streptomycin, kanamycin, amikacin, amphotericin B, benzylpenicillin, piperacillin, cefaloridine, cefalotin, cefazolin, cefamandole, cefotaxime, cefoxitin, cefmetazole, cefotetan and the like antibiotics, and glutathione, bucladesine sodium, pyrrocaineamide hydrochloride, vitamins and the like.

Among these drugs, arachidonic acid metabolites or derivatives thereof are suited for incorporating in liposomes, and prostaglandin is particularly desirable.

Prostaglandin is classified into groups A to J, and each group is further classified into subgroups 1 to 3. According to the present invention, prostaglandin E, especially prostaglandin E₁ is preferred.

Prostaglandin E is possessed of vasodilation, hypotention, gastric secretion inhibition, gastric movement acceleration, uterine contraction, diuresis, bronchodilation, bone resorption, immunosuppression and the like biological activities.

Prostaglandin E₁ is used for the (1) improvement of ulcers of extremities, necrosis and pain at the time of resting in chronic arterial obstruction (Buerger disease, chronic arteriosclerosis), (2) improvement of skin ulcers in progressive systemic sclerosis, systemic lupus erythematosus and diabetes mellitus, (3) improvement of vision disorders caused by peripheral circulation interruption in health hazard due to vibration and recovery of peripheral circulation, nervous and motor function disorders, (4) patency of arterial ducts in arterial duct dependent congenital heart disease, and (5) treatment of apareunia.

Next, the present invention is described with reference to inventive, reference and test examples, hut the invention is not restricted by them.

### BEST MODE OF CARRYING OUT THE INVENTION

### EXAMPLES

### Inventive Example 1. Preparation of prostaglandin E₁-incorporating sphingomyelin liposomal dispersion

Sphingomyelin in the amount shown in Formulation Example 1 was put into a glass container and firstly dissolved in chloroform. Next, prostaglandin E₁ (PGE₁) was dissolved in methanol, a portion of the solution equivalent to 1 mg of PGE₁ was put into the glass container and then the organic solvents were evaporated under a reduced pressure. Next, the aqueous solvent shown in Formulation Example 1 was added thereto, and the mixture was heated at about 60°C to effect enough hydration and then stirred using a vortex mixer to obtain a crude liposomal dispersion. Thereafter, the crude liposomal dispersion was extruded through a polycarbonate membrane filter having a pore size of 0.2 µm under a high pressure, thereby obtaining a liposomal dispersion. In the same manner, liposomal dispersions of Formulation Examples 2 to 11 were prepared.

| Formulation Example 1 | |
|---|---|
| PGE₁ | 10 mg |
| Bovine brain sphingomyelin | 0.8 g |
| (10 mM as lipid concentration) | |
| Glycerol | 2.6 g |
| Citric acid | 0.2 g |
| 1N Sodium hydroxide | proper amount (to adjust to pH 5.0) |
| Purified water (total volume) | 100 ml |

| Formulation Example 2 | |
|---|---|
| PGE₁ | 10 mg |
| Bovine brain sphingomyelin (10 mM as lipid concentration) | 0.8 g |
| Sucrose | 8.45 g |
| Glycerol | 0.3 g |
| Citric acid | 0.2 g |
| 1 N Sodium hydroxide | proper amount (to adjust to pH 5.0) |
| Purified water (total volume) | 100 ml |

| Formulation Example 3 | |
|---|---|
| PGE₁ | 10 mg |
| Bovine brain sphingomyelin (10 mM as lipid concentration) | 0.8 g |
| Lactose | 10 g |
| Citric acid | 0.2 g |
| 1 N Sodium hydroxide | proper amount (to adjust to pH 5.0) |
| Purified water (total volume) | 100 ml |

| Formulation Example 4 | |
|---|---|
| PGE₁ | 10 mg |
| Bovine brain sphingomyelin (20 mM as lipid concentration) | 1.6 g |
| Sucrose | 10 g |
| Citric acid | 0.2 g |
| 1 N Sodium hydroxide | proper amount (to adjust to pH 5.0) |
| Purified water (total volume) | 100 ml |

| Formulation Example 5 | |
|---|---|
| PGE₁ | 10 mg |
| Egg yolk sphingomyelin (10 mM as lipid concentration) | 0.8 g |
| Sucrose | 10 g |
| Citric acid | 0.2 g |
| 1 N Sodium hydroxide | proper amount (to adjust to pH 5.0) |
| Purified water (total volume) | 100 ml |

| Formulation Example 6 | |
|---|---|
| PGE₁ | 10 mg |
| Egg yolk sphingomyelin (10 mM as lipid concentration) | 0.8 g |
| Sucrose | 10 g |
| Citric acid | 0.05 g |
| 1 N Sodium hydroxide | proper amount (to adjust to pH 5.0) |
| Purified water (total volume) | 100 ml |

| Formulation Example 7 | |
|---|---|
| PGE₁ | 10 mg |
| Egg yolk sphingomyelin (1 mM as lipid concentration) | 0.08 g |
| Sucrose | 10 g |
| Citric acid | 0.05 g |
| 1 N Sodium hydroxide | proper amount (to adjust to pH 7.0) |
| Purified water (total volume) | 100 ml |

| Formulation Example 8 | |
|---|---|
| PGE₁ | 10 mg |
| Egg yolk sphingomyelin | 0.064 g |
| Cholesterol (1 mM as lipid concentration) | 0.016 g |
| Sucrose | 10 g |
| Citric acid | 0.05 g |
| 1 N Sodium hydroxide | proper amount (to adjust to pH 5.0) |
| Purified water (total volume) | 100 ml |

| Formulation Example 9 | |
|---|---|
| PGE₁ | 10 mg |
| Egg yolk sphingomyelin (0.01 mM as lipid concentration) | 0.008 g |
| Sucrose | 10 g |
| Citric acid | 0.05 g |
| 1 N Sodium hydroxide | proper amount (to adjust to pH 5.0) |
| Purified water (total volume) | 100 ml |

| Formulation Example 10 | |
|---|---|
| PGE₁ | 10 mg |
| Egg yolk sphingomyelin (0.001 mM as lipid concentration) | 0.0008 g |
| Sucrose | 10 g |
| Citric acid | 0.05 g |
| 1 N Sodium hydroxide | proper amount (to adjust to pH 5.0) |
| Purified water (total volume) | 100 ml |

| Formulation Example 11 | |
|---|---|
| PGE₁ | 10 mg |
| Egg yolk sphingomyelin (10 mM as lipid concentration) | 0.8 g |
| Glycerol | 2.62 g |
| Citric acid | 0.2 g |
| 1 N Sodium hydroxide | proper amount (to adjust to pH 5.0) |
| Purified water (total volume) | 100 ml |

### REFERENCE EXAMPLES

### Reference Example 1. Preparation of prostaglandin E₁-incorporating dipalmitoyl phosphatidylcholine or dimyristoyl phosphatidylcholine liposomal dispersion

The liposomal dispersions of Control Examples 1 to 8 were prepared in the same manner as described in Inventive Example 1, except that sphingomyelin was replaced by dipalmitoyl phosphatidylcholine or dimyristoyl phosphatidylcholine.

| Control Example 1 | |
|---|---|
| PGE₁ | 10 mg |
| Dipalmitoyl phosphatidylcholine (20 mM as lipid concentration) | 1.5 g |
| Sucrose | 10 g |
| Citric acid | 0.2 g |
| 1 N Sodium hydroxide | proper amount (to adjust to pH 5.0) |
| Purified water (total volume) | 100 ml |

| Control Example 2 | |
|---|---|
| PGE₁ | 10 mg |
| Dipalmitoyl phosphatidylcholine (10 mM as lipid concentration) | 0.73 g |
| Sucrose | 10 g |
| Citric acid | 0.2 g |
| 1 N Sodium hydroxide | proper amount (to adjust to pH 5.0) |
| Purified water (total volume) | 100 ml |

| Control Example 3 | |
|---|---|
| PGE₁ | 10 mg |
| Dipalmitoyl phosphatidylcholine (5 mM as lipid concentration) | 0.37 g |
| Sucrose | 10 g |
| Citric acid | 0.2 g |
| 1 N Sodium hydroxide | proper amount (to adjust to pH 5.0) |
| Purified water (total volume) | 100 ml |

| Control Example 4 | |
|---|---|
| PGE₁ | 10 mg |
| Dipalmitoyl phosphatidylcholine (1 mM as lipid concentration) | 0.073 g |
| Sucrose | 10 g |
| Citric acid | 0.2 g |
| 1 N Sodium hydroxide | proper amount (to adjust to pH 5.0) |
| Purified water (total volume) | 100 ml |

| Control Example 5 | |
|---|---|
| PGE₁ | 10 mg |
| Dimyristoyl phosphatidylcholine (20 mM as lipid concentration) | 1.4 g |
| Sucrose | 10 g |
| Citric acid | 0.2 g |
| 1 N Sodium hydroxide | proper amount (to adjust to pH 5.0) |
| Purified water (total volume) | 100 ml |

| Control Example 6 | |
|---|---|
| PGE₁ | 10 mg |
| Dipalmitoyl phosphatidylcholine (20 mM as lipid concentration) | 1.5 g |
| Sucrose | 10 g |
| Citric acid | 0.2 g |
| 1 N Sodium hydroxide | proper amount (to adjust to pH 4.0) |
| Purified water (total volume) | 100 ml |

| Control Example 7 | |
|---|---|
| PGE₁ | 10 mg |
| Dipalmitoyl phosphatidylcholine (20 mM as lipid concentration) | 1.5 g |
| Sucrose | 10 g |
| Citric acid | 0.2 g |
| 1 N Sodium hydroxide | proper amount (to adjust to pH 6.0) |
| Purified water (total volume) | 100 ml |

| Control Example 8 | |
|---|---|
| PGE₁ | 10 mg |
| Dipalmitoyl phosphatidylcholine (20 mM as lipid concentration) | 1.5 g |
| Sucrose | 10 g |
| Citric acid | 0.2 g |
| 1 N Sodium hydroxide | proper amount (to adjust to pH 7.0) |
| Purified water (total volume) | 100 ml |

### Test Example 1. Stability test

Each of the liposomal dispersions prepared in Inventive Example 1 was stored at 40°C for 1 month (except for the Control Example 8 dispersion which was stored at 40°C for 2 weeks), at -20°C of freezing for 3 months or at 50°C for 2 weeks after freeze-drying the prepared liposomal dispersion. After the preservation, determination of prostaglandin E₁ was carried out by the method shown in the following.

### 1-1. Determination of prostaglandin E₁

Extraction of prostaglandin E₁ from the liposomal dispersion was carried out in the following manner. A 1 ml portion of each of the liposomal dispersions containing prostaglandin E₁ was mixed with 1 ml of tetrahydrofuran and stirred and then further mixed with 2 ml of the elution solution for liquid chromatography use shown in the following and thoroughly stirred. This was centrifuged (5°C, 3,500 rpm, 15 minutes) to recover the supernatant to be used in the determination. The determination was carried out by a high performance liquid chromatography.

### Separation determination conditions

Mobile phase: 0.002 M phosphate buffer (pH 3.0)/acetonitrile mixture (63/37)
Column: a column packed with octadecylsilylated silica gel
Column temperature: 30°C
Flow rate: 1 ml/min
Sample injected: 50 µl
Measuring wave length: 200 nm

### 1-2. Test results

Table 1 shows results of the stability test on liposomal dispersions after their storage at 40°C for 1 month, liposomal dispersions after their storage at -20°C for 3 months and freeze-dried liposomal dispersions after their storage at 50°C for 2 weeks. The results were expressed as the residual ratio to the initial amount (%). In this case, higher values indicate superior stability.

**Table 1**

| | Preservation conditions | | |
|---|---|---|---|
| | Dispersion | Freeze drying | Freezing |
| Control Ex. 1. | 56.4 | 45.3 | - |
| Control Ex. 2. | 51.8 | 48.9 | - |
| Control Ex. 3. | 44.3 | 52.2 | - |
| Control Ex. 4. | 34.3 | 69.9 | - |
| Control Ex. 5. | 54.6 | 45.4 | - |
| Control Ex. 6. | 25.0 | 16.3 | - |
| Control Ex. 7. | 38.7 | 52.6 | - |
| Control Ex. 8. | 30.1 | 70.3 | - |
| Formulation Ex. 1. | 78.9 | - | - |
| Formulation Ex. 2. | 76.8 | - | - |
| Formulation Ex. 3. | 75.9 | 81.5 | - |
| Formulation Ex. 4. | 74.5 | 61.9 | - |
| Formulation Ex. 5. | 76.1 | 83.6 | - |
| Formulation Ex. 6. | 80.4 | 81.5 | - |
| Formulation Ex. 7. | - | 87.8 | - |
| Formulation Ex. 8. | - | 90.5 | - |
| Formulation Ex. 9. | - | 89.7 | - |
| Formulation Ex. 10. | - | 90.0 | - |
| Formulation Ex. 11. | - | - | 100.0 |

When the dispersions were preserved as such, stability of prostaglandin E₁ was excellent with a lipid concentration of 10 mM or more judging from the results of Control Examples 1 to 5. Also, in comparison with dipalmitoyl phosphatidylcholine and dimyristoyl phosphatidylcholine, the liposomal dispersions having sphingomyelin as the main component of the membrane-constituting lipids showed far superior stability.

When the dispersions were preserved under freeze-dried condition, stability of prostaglandin E₁ was excellent with a lipid concentration of 10 mM or less judging from the results of Control Examples 1 to 4. Also, in comparison with dipalmitoyl phosphatidylcholine and dimyristoyl phosphatidylcholine, the liposomal dispersions having sphingomyelin as the main component of the membrane-constituting lipids showed far superior stability.

On the basis of these results, it was found that the stability of drugs having poor stability in aqueous solution is improved when they are incorporated in liposomes whose main membrane-constituting lipid component is a sphingolipid which has sphingosine as the nucleus in its structure. Test Example 2. Encapsulation efficiency of prostaglandin E₁ in liposomes.

The prostaglandin E₁-incorporating liposomal dispersion described in Formulation Example 1 of Inventive Example 1 was diluted with phosphate-buffered saline (PBS) and then subjected to centrifugation (100,000 g, 1 hour, 4°C, centrifuge L8-M manufactured by Beckman). Encapsulation efficiency of prostaglandin E₁ in liposomes was calculated by determining prostaglandin E₁ in the resulting pellet by HPLC. As the result, the encapsulation efficiency was found to be 98.2%.

Based on this result, it was found that liposomes having sphingolipid as the main component of the membrane-constituting lipids can incorporate drugs at a sufficiently high ratio. It was suggested that such a high encapsulation efficiency in liposomes contributes to the stability of drugs which have poor stability in aqueous solution.

### INDUSTRIAL APPLICABILITY

Thus, the liposomes and liposomal dispersion of the present invention have sufficiently high encapsulation efficiency of drugs in liposomes and show excellent stability of drugs which have poor stability in aqueous solution. In addition, the liposomes and liposomal dispersion of the present invention can be prepared by an easy and simple procedure without requiring complex handling and therefore are suited for their industrial application.

## Claims

1. A liposomal preparation in which a drug is incorporated in liposomes comprised of a sphingolipid as the main component of the membrane-constituting lipids.

2. The liposomal preparation according to claim 1 wherein the sphingolipid is a sphingophospholipid.

3. The liposomal preparation according to claim 2 wherein the sphingophospholipid is sphingomyelin.

4. A liposomal dispersion in which the liposomes of any one of the claims 1 to 3 are dispersed.

5. The liposomal dispersion according to claim 4 wherein dispersion medium of the dispersion is an aqueous solvent.

6. The liposomal dispersion according to claim 5 wherein the aqueous solvent is a buffer solution.

7. The liposomal dispersion according to claim 6 wherein the buffer solution is a citrate buffer.

8. The liposomal dispersion according to claim 7 wherein concentration of the citrate buffer is from 0.05 to 0.2% by weight.

9. The liposomal dispersion according to any one of the claims 4 to 8 wherein concentration of the sphingolipid is 10 mM or more.

10. The liposomal dispersion according to claim 9 wherein concentration of the sphingolipid is from 10 to 40 mM.

11. The liposomal dispersion according to any one of the claims 4 to 10 wherein pH of the dispersion is from 5 to 7.

12. The liposomal dispersion according to any one of the claims 4 to 11 wherein it can be preserved under dispersed state, dried state or frozen state.

13. The liposomal preparation or liposomal dispersion according to any one of the claims 1 to 12 wherein the drug is a drug which has poor stability in its aqueous solution.

14. The liposomal preparation or liposomal dispersion according to any one of the claims 1 to 12 wherein the drug is an arachidonic acid metabolite or a derivative thereof.

15. The liposomal preparation or liposomal dispersion according to any one of the claims 1 to 12 wherein the drug is a prostaglandin.

16. The liposomal preparation or liposomal dispersion according to claim 15 wherein the prostaglandin is prostaglandin E₁.

17. A liposomal dispersion prepared by dispersing liposomes which are comprised of sphingomyelin as the main component of the membrane-constituting lipids and incorporating prostaglandin E₁ therein, wherein concentration of sphingomyelin is from 10 to 40 mM, the dispersion medium is a citrate buffer of from 0.05 to 0.2% by weight, the pH is from 5 to 7 and mean particle size of the liposomes is from 100 to 250 nm.
